Europäisches Patentamt

European Patent Office

Office européen des brevets

(11) Veröffentlichungsnummer : **0 407 804 B1**

(12) **EUROPÄISCHE PATENTSCHRIFT**

(45) Veröffentlichungstag der Patentschrift :
**03.11.93 Patentblatt 93/44**

(21) Anmeldenummer : **90112105.3**

(22) Anmeldetag : **26.06.90**

(51) Int. Cl.⁵ : $A61K\ 31/28$, $A61K\ 47/10$,
$A61K\ 47/26$, $A61K\ 9/14$

(54) **Wasserlösliche pharmazeutische Metallocen-Komplex-Zusammensetzung.**

(30) Priorität : **14.07.89 DE 3923270**

(43) Veröffentlichungstag der Anmeldung :
**16.01.91 Patentblatt 91/03**

(45) Bekanntmachung des Hinweises auf die
Patenterteilung :
**03.11.93 Patentblatt 93/44**

(84) Benannte Vertragsstaaten :
**AT BE CH DE DK ES FR GB GR IT LI LU NL SE**

(56) Entgegenhaltungen :
**EP-A- 0 186 505**
**EP-A- 0 202 673**
**EP-A- 0 216 362**
**WO-A-83/00868**
**DE-A- 2 923 334**
**CHEMICAL REVIEWS, Band 87, Nr. 5, Oktober**
**1987, Washington, DC (US); P. KÖPF-MAIER et**
**al., Seiten 1137-1152**

(73) Patentinhaber : **Medac Gesellschaft für**
**klinische Spezialpräparate mbH**
**Fehlandtstrasse 3, Postfach 30 36 29**
**D-20312 Hamburg (DE)**

(72) Erfinder : **Müller, Bernd, Prof.Dr.**
**Schlotfeldtsberg 14 A**
**D-2302 Flintbek (DE)**
Erfinder : **Lucks, Stefan**
**Deliusstrasse 23a**
**D-2300 Kiel 1 (DE)**
Erfinder : **Müller, Rainer, Priv.-Doz. Dr.**
**Samwerstrasse 8**
**D-2300 Kiel 1 (DE)**
Erfinder : **Mohr, Wilfried**
**Oelsnerring 75**
**D-2000 Hamburg 52 (DE)**

(74) Vertreter : **UEXKÜLL & STOLBERG**
**Patentanwälte**
**Beselerstrasse 4**
**D-22607 Hamburg (DE)**

## Beschreibung

Die Erfindung betrifft wasserlösliche pharmazeutische Metallocen-Komplex-Zusammensetzungen, die als Cytostatikum in der Krebstherapie eingesetzt werden können.

Die Verwendung von Metallocen-Komplexen als Cytostatika ist aus DE-A-29 23 334 und DE-A-35 18 447 sowie Chem. Rev. **87**, 1137-1152 (1987) bekannt. Die Verbindungen sind wegen der lipophilen Cyclopentadienyl-Reste sehr schwer wasserlöslich und erweisen sich zudem als relativ instabil in wäßriger Lösung, so daß für die parenterale Verabreichung in Form einer Injektionslösung bislang geeignete Lösungen fehlen.

In Dokl. Akad. Nauk SSR **266**, 883 (1982) werden wasserlösliche Vanadocenacylate beschrieben, die durch Umsetzung von Vanadocen mit Hydroxypropantricarbonsäure hergestellt werden. Diese Arbeitsweise ist auf die genannten Anionen beschränkt.

In der DE-A-29 23 334 wird die Verwendung von Dimethylsulfoxid als Lösungsvermittler für Titanocene beschrieben. Das Dimethylsulfoxid ist aufgrund seiner pharmakodynamischen Eigenwirkung und seiner Embryotoxizität jedoch nachteilig.

Weiter ist aus einer Veröffentlichung von K. Döppert in J. Organomet. Chem. **319**, 351 (1987) bekannt, daß Titanocen-Komplexe in wäßrigen Lösungen hydrolytisch zu unlöslichen Polymeren abgebaut werden. Danach erschien es nicht möglich, Metallocen-Komplexe in eine wasserlösliche Form zu überführen, aus der für eine parenterale Verabreichung genügend stabile Injektionslösungen mit therapeutisch ausreichenden Konzentrationen an Metallocen-Komplex hergestellt werden können.

Die aus EP-A-0 186 505 und EP-A-0 216 362 bekannten, zur Stabilisierung instabiler Organometallverbindungen eingesetzten Polyole fanden bislang keine Verwendung zur Löslichmachung in Wasser schwerlöslicher Metallocen-Komplexe, insbesondere Titanocen-Komplexe.

Der Erfindung liegt die Aufgabe zugrunde, eine physiologisch verwendbare wasserlösliche Metallocen-Komplex-Zusammensetzung, die in Form einer wäßrigen Lösung mit therapeutisch ausreichender Konzentration an Metallocen-Komplex genügend stabil ist, um parenteral verabreicht werden zu können, und ein Verfahren zur Herstellung dieser Zusammensetzung zur Verfügung zu stellen. Insbesondere sollen die Lösungen aufgrund einer wesentlich erhöhten Lösungsgeschwindigkeit der Zusammensetzung unmittelbar vor der Anwendung bequem herstellbar sein. Derartige Lösungen sind insbesondere als Cytostatika für die Krebstherapie von Bedeutung.

Diese Aufgabe wird überraschenderweise erfindungsgemäß durch eine Metallocen-Komplex-Zusammensetzung gelöst, die durch Mischen von einem Metallocen-Komplex, einem Polyol, Wasser und gegebenenfalls Zusatzstoffen und anschließendes Entfernen des Wassers aus der Mischung erhältlich ist.

Die erfindungsgemäße wasserlösliche pharmazeutische Metallocen-Komplex-Zusammensetzung ist insbesondere erhältlich durch Mischen von

0,01 bis 2 Gew.% Metallocen-Komplex,

0,1 bis 20 Gew.% eines Polyols aus der Gruppe Glykole, Zuckeralkohole, Kohlenhydrate oder deren Mischungen,

58 bis 99,89 Gew.% Wasser und

0 bis 20 Gew.% Zusatzstoffe

und anschließendes Entfernen des Wassers.

Ganz besonders bevorzugt ist die erfindungsgemäße Metallocen-Komplex-Zusammensetzung dadurch erhältlich, daß

0,02 bis 0,4 Gew.% Metallocen-Komplex, 0,5 bis 6,0 Gew.% Polyol, 91,6 bis 99,48 Gew.% Wasser und 0 bis 3,0 Gew.% Zusatzstoffe gemischt werden.

Geeignete Metallocen-Komplexe entsprechen der Formel $(Cp)_2M^nX_{n-2}$, in der Cp das Cyclopentadienylanion, M ein Übergangsmetall mit der Wertigkeit n und X ein ein- oder mehrwertiges Anion ist. Bevorzugt entsprechen die eingesetzten Komplexe der Formel $Cp_2M^{IV}X_2$. X kann insbesondere ein Halogenid, z.B. ein Chlorid, aber auch ein anderes Anion sein.

Bevorzugt finden Vanadocen-, Hafnocen-, Zirkonocen-, Molybdänocen-, sowie Tantalocen-Komplexe und/oder deren Mischungen und besonders bevorzugt Titanocen-Komplexe, insbesondere Titanocen-dichlorid Verwendung. Unter dem Begriff Metallocen-Komplexe werden sowohl Metallocen-Verbindungen, die keine Substitutenten an den beiden Cyclopentadien-Ringen aufweisen, als auch solche verstanden, die an mindestens einem der Cyclopentadien-Ringe substituiert sind.

Als Polyole werden bevorzugt Glykole, Zuckeralkohole, Kohlenhydrate oder deren Mischungen, besonders bevorzugt Glycerin, 1,2-Propylenglykol, 1,5-Pentandiol, Polyethylenglykole, Blockcopolymere aus Propylenglykol und Ethylenglykol, Pentaerythritol, Glucose, Fructose, Saccharose (Sucrose), Lactose oder deren Mischungen und ganz besonders bevorzugt Sucrose, Lactose, Glucose, Mannitol, Sorbitol oder deren Mischungen eingesetzt. Bevorzugt sind Polyole mit einer Glasübergangstemperatur $T_g$ im Bereich von etwa -30°C bis -50°C.

Als Hilfsstoff findet insbesondere Natriumchlorid als Isotonisierungsmittel, bevorzugt in Mengen von etwa 0,9 Gew.%, Verwendung.

Bei der Herstellung der erfindungsgemäßen Zusammensetzung werden Metallocen-Komplex, Polyol, Wasser und gegebenenfalls eingesetzte Zusatzstoffe gemischt und anschließend das in dieser Mischung enthaltene Wasser, bevorzugt mittels Gefriertrocknung, entfernt. Zur beschleunigten Auflösung bzw. Dispergierung des Metallocen-Komplexes in der wäßrigen Lösung wird die Mischung bevorzugt mit Ultraschall beschallt. Gegebenenfalls kann die Auflösung auch unter Erwärmen erfolgen. Für eine großtechnische Herstellung der erfindungsgemäßen Zusammensetzung werden anstelle der Ultraschallbehandlung Cosolventien wie z.B. Dimethylsulfoxid und Tetrahydrofuran, bevorzugt in Konzentrationen von 0,5 bis 10 Gew.%, eingesetzt. Sie beschleunigen die Auflösung bzw. das Dispergieren des Metallocen-Komplexes in der wäßrigen Lösung. Allerdings muß bei Verwendung von Cosolventien berücksichtigt werden, daß sich die eutektische Temperatur der Mischung und damit die Einfriertemperatur sowie die Parameter des Gefriertrocknungsprozesses verändern. Durch die Gefriertrocknung werden die Cosolventien zusammen mit dem Wasser entfernt, so daß die erfindungsgemäße Metallocen-Komplex-Zusammensetzung als lyophilisierte Trockensubstanz zurück bleibt. Sie ist nach erneuter Wasserzugabe unter Bildung einer klaren, für eine parenterale Verabreichung genügend stabilen Lösung vollständig wieder auflösbar. Der Metallocen-Komplex ist in ihr bevorzugt in Konzentrationen von 0,05 bis 5 mg/ml $H_2O$ enthalten. Die erhöhte Stabilität der wäßrigen Lösung der erfindungsgemäßen Zusammensetzung gegenüber der wäßrigen Lösung von Metallocen-Komplexen zeigt sich in einer erheblich später eintretenden Trübung infolge der Bildung von Di- und Oligomeren. Damit steht eine wasserlösliche pharmazeutische Metallocen-Komplex-Zusammensetzung zur Verfügung, die in Form ihrer wäßrigen, therapeutische Konzentrationen an Metallocen-Komplex enthaltenden Lösung ausreichend stabil ist, um bequem parenteral als Cytostatikum in der Krebstherapie appliziert werden zu können.

Die Erfindung wird in den folgenden Beispielen näher erläutert.

Beispiel 1:

    20,0 mg Titanocendichlorid
    90,0 mg Natriumchlorid
    100,0 mg Mannitol
    10,0 ml (Wasseraqua pro injectione)

Die oben angegebenen Bestandteile wurden gemischt, die Mischung wurde ca. 1 Stunde lang mit Ultraschall beschallt, durch ein 0,45 μm Membranfilter filtriert und bei -50°C in einem Injektionsfläschchen eingefroren. Nach der sich anschließenden den Gefriertrocknung bei -35°C konnte das zurückgebliebene Lyophilisat vollständig in Wasser unter Bildung einer stabilen, klaren Lösung aufgelöst werden. Die Konzentration des Wirkstoffes in dieser Lösung betrug 2 mg Wirkstoff pro 1 ml Lösung.

Beispiel 2:

    10 mg Titanocendichlorid
    45 mg Natriumchlorid
    255 mg Sorbitol
    5 ml (Wasseraqua pro injectione)

Die Herstellung erfolgte wie unter Beispiel 1 beschrieben. Die durch Auflösung des Lyophilisates in 5 ml Wasser erhaltene Lösung enthielt den Wirkstoff in einer Konzentration von 2 mg/ml.

Beispiel 3:

    10 mg Titanocendichlorid
    255 mg Sorbitol
    5 ml Wasser (aqua pro injectione)

Die Herstellung erfolgte wie unter Beispiel 1 beschrieben. Das nach der Gefriertrocknung zurückgebliebene Lyophilisat ergab mit 5 ml Wasser eine klare, isotone Lösung mit 2 mg Wirkstoff pro ml Lösung.

Beispiel 4:

10 mg Titanocendichlorid
100 mg Dimethylsulfoxid
255 mg Sorbitol
5 ml Aqua pro injectione

Nach Mischen und Auflösen der oben angegebenen Komponenten wurde die erhaltene Lösung durch ein 0,45 µm Membranfilter filtriert, bei -70°C eingefroren und anschließend gefriergetrocknet. Dabei wurde neben dem Wasser auch das Cosolvens Dimethylsulfoxid entfernt. Das erhaltene Lyophilisat war in 5 ml Wasser vollständig löslich und bildete eine isotone, stabile Lösung mit einem Wirkstoffgehalt von 2 mg/ml.

Beispiel 5:

Es wurden Lösungen folgender Zusammensetzung hergestellt:
2 mg Titanocen-dichlorid
51 mg Polyol
10 ml Wasser (aqua pro injectione)
Als Polyol fanden Verwendung:
A. Sucrose
B. Lactose
C. Glucose
Folgende Bedingungen wurden für die Gefriertrocknung angewendet:

| | Temperatur | Druck |
|---|---|---|
| A | −37°C | $< 2 \cdot 10^{-1}$ mbar |
| B | −35°C | $< 2.5 \cdot 10^{-1}$ mbar |
| C | −50°C | $< 3.9 \cdot 10^{-2}$ mbar |

Nach Enfernen des größten Teils des Wassers, was nach mehreren Stunden erreicht ist, wird das Vakuum langsam reduziert und gleichzeitig die Temperatur um Schritte von 7°C bis auf 20°C angehoben; erforderlichenfalls kann zur vollständigen Trocknung bis auf 40°C gegangen werden. Es wurden weitgehend wasserfreie Lyophilisate erhalten, die sich leicht in Wasser lösen lassen.

Beispiel 6

Das Beispiel 3 wurde wiederholt, wobei jedoch folgende Metallocen-Komplexverbindungen eingesetzt wurden:
A. 10 mg Zirkonocen-dichlorid (weißes kristallines Pulver)
B. 10 mg Hafnocen-dichlorid (weißes kristallines Pulver, das zur Klumpenbildung neigt)
C. 10 mg Molybdenocen-dichlorid (schwarz-braunes feinkörniges Pulver)
D. 10 mg Vanadocen-dichlorid (dunkelgrünes kristallines Pulver)
E. 10 mg Vanadocen-dichlorid + 10 mg Ascorbinsäure
Auf die Proben A bis E wurden 5 g isotonische Sorbitollösung (entsprechend 255 mg Sorbitol im Lyophilisat) gegeben.

Probe A löste sich in der Sorbitollösung bei Raumtemperatur nach kurzem Schütteln auf und ergab eine farblose Lösung.

Probe B war weniger rasch löslich, die Auflösung wurde durch Ultraschall unterstützt. Dabei löste sich die Substanz klar und farblos.

Probe C war mäßig schnell löslich, wobei eine Ultraschallbehandlung die Auflösung erheblich erleichtert. Das dunkle Pulver bildete zunächst eine schmutzig braune Suspension, die nach einiger Zeit (3 bis 4 Min.) in eine dunkelgrüne klare Lösung überging.

Die Proben D und E ähnelten in ihren Lösungseigenschaften dem Titanocen-dichlorid. Zur Zerkleinerung von Klumpen wurde auch hier mit Ultraschall behandelt. Eine Wärmezufuhr erleichterte den Lösungsvorgang erheblich. Das Vanadocendichlorid löste sich klar mit dunkelgrüner Farbe in der Sorbitollösung auf.

Nach dem Lösen wurden die Probengefäße im Gefrierschrank bei -18°C eingefroren und danach im Gefriertrockner zunächst bei -35°C 24 Stunden lang durchgefroren und anschließend 4 Tage lang getrocknet.

Während der Gefriertrocknung traten bei den Proben A und B keine Komplikationen auf. Es bildete sich in beiden Fällen ein lockeres weißes Lyophilisat.

Bei der Trocknung von Probe C (Molybdenocen-dichlorid) veränderte sich die Farbe von dunkelgrün nach beige. Es bildete sich ein lockeres Lyophilisat.

Bei der Gefriertrocknung der Probe E (mit Ascorbinsäure) traten bei einer Temperatur von -35°C an der Oberfläche Anschmelzungen auf. Die Trocknung wurde trotzdem weitergeführt. Die entstehenden Lyophilisate der Proben D und E waren grün gefärbt. Die angeschmolzenen Stellen erschienen etwas dunkler.

Die Lyophilisate der Proben A und B zeigten eine hervorragende Löslichkeit. Nach Zugabe von Wasser war nach kurzem Schütteln (max. 30 Sek.) eine klare farblose Lösung erhältlich. Das Lyophilisat der Probe C mußte etwa 5 Min. lang mit Wasser intensiv geschüttelt werden, um eine klare Lösung zu erhalten. Dabei ging das beige gefärbte Lyophilisat langsam in eine olivgrüne Lösung über. Eine frisch hergestellte Vergleichslösung mit Molybdenocen-dichlorid zeigte dieselbe Farbe.

Das Lyophilisat des Vanadocen-dichlorids war in seiner Lösungsgeschwindigkeit dem Titanocen-dichlorid recht ähnlich, unabhängig von der Abwesenheit oder Anwesenheit von Ascorbinsäure. Nach Zugabe von Wasser mußte ungefähr 2 Min. lang geschüttelt werden, um eine klare dunkelgrüne Lösung zu erhalten.

Die jeweils aus den Lyophilisaten gebildeten Lösungen erwiesen sich stabil und waren für eine parenterale Verabreichung geeignet.

**Patentansprüche**

1. Pharmazeutische Metallocen-Komplex-Zusammensetzung mit erhöhter Löslichkeit und Lösungsgeschwindigkeit sowie verbesserter Stabilität, **dadurch gekennzeichnet,** daß sie durch Mischen von einem Metallocen-Komplex, einem Polyol, Wasser und gegebenenfalls Zusatzstoffen und anschließendes Entfernen des Wassers aus der Mischung erhältlich ist.

2. Zusammensetzung nach Anspruch 1, **dadurch gekennzeichnet,** daß sie durch Verwendung von 0,01 bis 2 Gew.% Metallocen-Komplex, 0,1 bis 20 Gew.% Polyol, 58 bis 99,89 Gew.% Wasser und 0 bis 20 Gew.% Zusatzstoffen erhältlich ist.

3. Zusammensetzung nach einem der Ansprüche 1 oder 2, **dadurch gekennzeichnet,** daß sie durch Verwendung von 0,02 bis 0,4 Gew.% Metallocen-Komplex, 0,5 bis 6,0 Gew.% Polyol, 91,6 bis 99,48 Gew.% Wasser und 0 bis 3,0 Gew.% Zusatzstoffen erhältlich ist.

4. Zusammensetzung nach einem der Ansprüche 1 bis 3, **dadurch gekennzeichnet**, daß sie durch Verwendung von Titanocen-, Tantalocen-, Hafnocen-, Zirkonocen-, Molybdänocen-, Vanadocen-Komplexen oder deren Mischungen als Metallocen-Komplex erhältlich ist.

5. Zusammensetzung nach Anspruch 4, **dadurch gekennzeichnet,** daß sie durch Verwendung von Titanocendichlorid als Metallocen-Komplex erhältlich ist.

6. Zusammensetzung nach einem der Ansprüche 1 bis 5, **dadurch gekennzeichnet,** daß sie durch Verwendung von Glykolen, Zuckeralkoholen, Kohlenhydraten oder deren Mischungen als von Glykolen, Zuckeralkoholen, Kohlenhydraten oder deren Mischungen als Polyol erhältlich ist.

7. Zusammensetzung nach Anspruch 6, **dadurch gekennzeichnet,** daß sie durch Verwendung von Glycerin, 1,2-Propylenglykol, 1,5-Pentandiol, Polyethylenglykolen, Blockcopolymeren aus Propylenglykol und Ethylenglykol, Pentaerythritol, Sorbitol, Mannitol, Glucose, Fructose, Saccharose (Sucrose), Lactose oder deren Mischungen als Polyol erhältlich ist.

8. Zusammensetzung nach einem der Ansprüche 1 bis 7, **dadurch gekennzeichnet,** daß sie durch Verwendung von Natriumchlorid als Zusatzstoff erhältlich ist.

9. Verfahren zur Herstellung der Zusammensetzung nach einem der Ansprüche 1 bis 8, **dadurch gekennzeichnet,** daß die Entfernung des Wassers durch Gefriertrocknung der Mischung vorgenommen wird.

10. Verfahren nach Anspruch 9, **dadurch gekennzeichnet,** daß das Lösen der Komponenten unter Beschallung mit Ultraschall und/oder unter Verwendung eines Cosolvens und/oder unter Erwärmen durchgeführt wird.

EP 0 407 804 B1

**11.** Verfahren nach Anspruch 10, **dadurch gekennzeichnet,** daß als Cosolvens Dimethylsulfoxid, Tetrahydrofuran und/oder deren Mischungen eingesetzt werden.

**12.** Verwendung einer Zusammensetzung nach einem der Ansprüche 1 bis 8 zur Herstellung einer Injektionslösung zur Verwendung als Cytostatikum in der Krebstherapie.

## Claims

1. Pharmaceutical composition containing metallocene complex with increased solubility and rate of dissolution and improved stability, **characterised in that** it is obtainable by mixing a metallocene complex, a polyol, water and optionally additives, and subsequently removing the water from the mixture.

2. Composition according to claim 1, **characterised in that** it is obtainable by using 0.01 to 2% by wt. of metallocene complex, 0.1 to 20% by wt. of polyol, 58 to 99.89% by wt. of water and 0 to 20% by wt. of additives.

3. Composition according to one of claims 1 or 2, **characterised in that** it is obtainable by using 0.02 to 0.4% by wt. of metallocene complex, 0.5 to 6.0% by wt. of polyol, 91.6 to 99.48% of water and 0 to 3.0% by wt. of additives.

4. Composition according to one of claims 1 to 3, **characterised in that** it is obtainable by using titanocene, tantalocene, hafnocene, zirconocene, molybdenocene, vanadocene complexes or mixtures thereof as metallocene complex.

5. Composition according to claim 4, **characterised in that** it is obtainable by using titanocene dichloride as metallocene complex.

6. Composition according to one of claims 1 to 5, **characterised in that** it is obtainable by using glycols, sugar alcohols, carbohydrates or mixtures thereof as polyol.

7. Composition according to claim 6, **characterised in that** it is obtainable by using glycerol, 1,2-propylene glycol, 1,5-pentane diol, polyethylene glycols, block copolymers of propylene glycol and ethylene glycol, pentaerythritol, sorbitol, mannitol, glucose, fructose, saccharose (sucrose), lactose or mixtures thereof as polyol.

8. Composition according to one of claims 1 to 7, **characterised in that** it is obtainable by using sodium chloride as additive.

9. Process for the preparation of the composition according to one of claims 1 to 8, **characterised in that** the removal of the water is carried out by freeze-drying the mixture.

10. Process according to claim 9, **characterised in that** the dissolution of the components is carried out with exposure to ultrasonic waves and/or with the use of a cosolvent and/or with heating.

11. Process according to claim 10, **characterised in that** dimethyl sulphoxide, tetrahydrofuran and/or mixtures thereof are used as cosolvent.

12. Use of a composition according to one of claims 1 to 8 for the preparation of an injection solution for use as a cytostatic agent in cancer therapy.

## Revendications

1. Composition pharmaceutique à base de complexe de métallocène ayant une solubilité et une vitesse de solubilisation plus élevées ainsi qu'une stabilité améliorée, caractérisée en ce qu'on peut l'obtenir en mélangeant un complexe de métallocène, un polyol, de l'eau et, éventuellement, des additifs, et en éliminant ensuite l'eau du mélange.

2. Composition selon la revendication 1, caractérisée en ce qu'on peut l'obtenir en utilisant de 0,01 à 2% en poids de complexe de métallocène, de 0,1 à 20% en poids de polyol, de 58 à 99,89% en poids d'eau et de 0 à 20% en poids d'additifs.

3. Composition selon l'une des revendications 1 ou 2, caractérisée en ce qu'on peut l'obtenir en utilisant de 0,02 à 0,4% en poids de complexe de métallocène, de 0,5 à 6% en poids de polyol, de 91,6 à 99,48% en poids d'eau et de 0 à 3,0% en poids d'additifs.

4. Composition selon l'une des revendications 1 à 3, caractérisée en ce qu'on peut l'obtenir en utilisant des complexes de titanocène, tantalocène, hafnocène, zirconocène, molybdénocène, vanadocène ou des mélanges de ceux-ci, comme complexe de métallocène.

5. Composition selon la revendication 4, caractérisée en ce qu'on peut l'obtenir en utilisant du dichlorure de titanocène comme complexe de métallocène.

6. Composition selon l'une des revendications 1 à 5, caractérisée en ce qu'on peut l'obtenir en utilisant des glycols, des sucre-alcools, des glucides ou des mélanges de ceux-ci, comme polyol.

7. Composition selon la revendication 6, caractérisée en ce qu'on peut l'obtenir en utilisant de la glycérine, du 1,2-propylène-glycol, du 1,5-pentanediol, des polyéthylène-glycols, des copolymères statistiques de propylène-glycol et d'éthylène-glycol, du pentaérythritol, du sorbitol, du mannitol, du glucose, du fructose, du saccharose (sucrose), du lactose ou des mélanges de ceux-ci, comme polyol.

8. Composition selon l'une des revendications 1 à 7, caractérisée en ce qu'on peut l'obtenir en utilisant du chlorure de sodium comme additif.

9. Procédé pour préparer la composition selon l'une des revendications 1 à 8 , caractérisé en ce qu'on effectue l'élimination de l'eau par lyophilisation du mélange.

10. Procédé selon la revendication 9, caractérisé en ce qu'on effectue la dissolution des composants par exposition à l'action des ultra-sons et/ou par utilisation d'un cosolvant et/ou par chauffage.

11. Procédé selon la revendication 10, caractérisé en ce qu'on utilise du diméthylsulfoxyde, du tétrahydrofuranne et/ou des mélanges de ceux-ci comme cosolvant.

12. Utilisation d'une composition selon l'une des revendications 1 à 8 pour préparer une solution injectable à utiliser comme agent cytostatique lors du traitement du cancer.